Europäisches Patentamt

European Patent Office

Office européen des brevets

(19)

(11) Publication number: 0 325 426
A1

# EUROPEAN PATENT APPLICATION

(12)

(21) Application number: 89300441.6

(22) Date of filing: 18.01.89

(51) Int. Cl.⁴: **A 61 B 17/34**
G 01 N 24/02, A 61 B 10/00

(30) Priority: 19.01.88 US 145143   25.11.88 US 275894

(43) Date of publication of application:
26.07.89 Bulletin 89/30

(84) Designated Contracting States:
AT BE CH DE ES FR GB GR IT LI NL SE

(71) Applicant: Lufkin, Robert Burnham
12961 Mulholland Drive
Beverly Hills California 90210 (US)

Zwarun, Andrew Alexander
10 Schoolhouse Lane Roslyn Heights
New York New York 11577 (US)

(72) Inventor: Lufkin, Robert Burnham
12961 Mulholland Drive
Beverly Hills California 90210 (US)

Zwarun, Andrew Alexander
10 Schoolhouse Lane Roslyn Heights
New York New York 11577 (US)

(74) Representative: Jennings, Nigel Robin et al
KILBURN & STRODE 30 John Street
London WC1N 2DD (GB)

(54) Magnetic resonance needle.

(57) An implement such as a biopsy needle (10) is formed of an alloy which has at least 35% and preferably about 49% nickel content. When the alloy is used to form a needle the high nickel content of the allow creates an artifact of a size to provide a sufficient needle image for localisation without obscuring the underlying anatomy.

EP 0 325 426 A1

Bundesdruckerei Berlin

Description

# MAGNETIC RESONANCE NEEDLE

## BACKGROUND OF THE INVENTION

The present invention relates to Magnetic Resonance (MR) Compatible Products, and more particularly, to a biopsy needle which is MR compatible.

Magnetic Resonance Imaging (MRI) is a non-invasive method of mapping and examining the internal structure of the body. It avoids the use of ionizing radiation which is used in some other non-invasive mapping methods. MRI uses radio frequency radiation in the presence of a controlled magnetic field to produce high quality cross-sectional images of the body. Currently, most MRI is high-resolution, thin-section imaging.

Because of the strong magnetic field used with MRI, substances placed therein, depending upon their own magnetic properties, can be affected or can affect the field or the image produced. If a substance is placed in a magnetic field, induced magnetization in the substance is added to that applied field. The ratio of induced magnetism to that of the applied field is termed magnetic susceptibility.

Most materials have negative susceptibility and are not magnetically very active. Some materials, on the other hand, are magnetically active. For example, paramagnetic, superparamagnetic, and ferromagnetic substances, characterized by the predominant effect of unpaired electron spins, have positive susceptibilities. In materials as complex as biological tissues, a range of magnetic susceptibilities is found. The spread in magnetization through the tissue causes a number of MR relaxation phenomena.

One problem that can occur in MRI with materials having positive susceptibilities is the creation of artifacts which can cause severe degradation, or other, more subtle effects that can lead to misinterpretation of the MR image. Another problem that occurs with such material is deflection in the magnetic field.

Needle aspiration biopsy is a safe, reliable technique for evaluation of various masses. In order to more efficiently perform a needle aspiration biopsy an imaging technique is used to guide the aspiration biopsy needle to the correct position. It is known in the art to use biopsy needles in conjunction with computerized tomography (CT). The CT scan allows the person performing the procedure to direct and guide the biopsy needle.

Although MRI is a safe and well accepted imaging technique, it has not been considered appropriate for guided biopsies. Conventional biopsy needles, which have positive susceptibilities, are not MR compatible because of interactions between the needle and the magnetic field. As aforementioned, artifacts are created by these conventional needles. These artifacts can obscure anatomic detail or prevent needle tip localization.

It has been found in the prior art that when conventional needles are usd with magnetic resonance the size of the artifact formed makes the needle tip look bigger than it actually is. Thus, while it may be easier to locate the needle tip on the image, the large size of the artifact makes exact location of the tip difficult, and further, such large artifact obscures underlying anatomy. Additionally, due to deflection in the magnetic field, conventional needles may move in the magnetic field which is potentially dangerous.

Most standard medical aspiration needles are made of stainless steel alloys which include chrome and nickel. It is known in the art that when nickel containing needles are used in connection with MR imaging the artifacts produced are inversely proportional in size to the percentage of nickel in the needle. It has been conventional technique to locate the needle itself by visualizing the needle tip image and thus, although it has been desired to avoid large artifact, it has also been considered desirable to avoid too small an artifact because it was felt that too small an artifact would not provide sufficient visibility in the MR image. Thus, in a 1986 study by J.T. Ferruci, P.R. Mueller, D.D. Stark, J. Wittenberg, J.F. Simone, R.J. Butch, and P. Beaulieu entitled "A Non-Ferromagnetic Needle for MR Guided Aspiration Biopsy: Design and Clinical Trials" Abstract-Society of Magnetic Resonance In Medicine Vol. 4, 8/22/86 it was found that a needle having 9% nickel content gave excessive artifact while a needle with 35% nickel gave no artifact. Mueller et al selected a needle with 12% nickel for further investigation.

Image degrading artifacts and deflection are created by ferromagnetic objects other than needles. Examples of such objects are surgical clips, infusion sets, wires, drains, electrodes and staples.

Depending on the quality, shape and magnetic susceptibility of the various objects used with MR, image distortion and movement in the magnetic field may occur. For example, the deflection that occurs with conventional stainless steel winged infusion sets could present a problem for a patient in the MR environment. Situations where winged infusion sets are used often involve precarious placement of the needle in a vessel where magnetic field induced motion could dislodge the needle tip or result in infiltration.

Accordingly, it is an object of the present invention to provide an implement which can be used in conjunction with magnetic resonance.

It is a further object of this invention to provide such an implement which is MR compatible and which does not create MR image degrading artifacts.

Yet a further object of this invention is to provide such an implement which will minimize the force of deflection in a magnetic field and which will not move in the magnetic field.

It is yet a further objective of this invention to provide an aspiration biopsy needle which produces appropriate artifact such as to allow needle localization without obscuring underlying anatomy.

## BRIEF DESCRIPTION

In one embodiment of the invention, an aspiration biopsy needle for use with magnetic resonance is provided. The needle is formed of an alloy which includes about forty-nine percent (49%) by weight nickel; between 15.00 - 17.00% by weight molybdenum; between 14.50 - 16.50% chromium; between 4.00 - 7.00% iron; and between 3.00 - 4.50% tungsten.

## BRIEF DESCRIPTION OF THE DRAWINGS

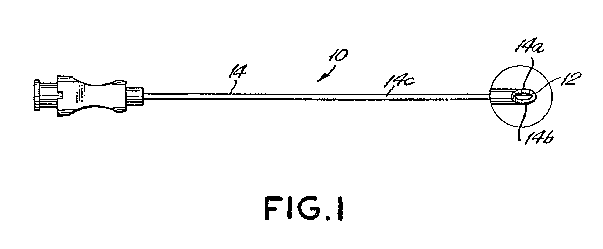

Fig. 1 is an enlarged sectional view of an aspiration biopsy needle made in accordance with the teachings of the present invention.

## DESCRIPTION OF THE PREFERRED EMBODIMENT

Referring now to the drawings and more particularly to Fig. 1, the reference numeral 10 denotes an aspiration biopsy needle. Needle 10 includes a tip 12 and a tubular body 14. Tubular body 14 has an outer wall 14a and an inner wall 14b which together define an interior channel 14c.

Biopsy needle 10 is designed to be used with high resolution MR scanners from 0.02 to 2.0 Tesla, such as the 0.3 Tesla MR Scanner, fonar B-3000M, made by Fonar Corporation of Melville, New York.

Biopsy needle is made from alloy C-276, (available from Superior Tube in Collegeville, Pennsylvania). Alloy C-276 is a high-temperature strength alloy with the following preferred weight percentage composition: about 49% nickel; about 15.00 - 17.00% molybdenum; about 14.50 - 16.50% chromium; about 4.00 - 7.00% iron; about 3.00 - 4.50% tungsten; up to about .08% carbon; up to about 1.00% magnesium; up to about 1.00% silicon; up to about 2.50% cobalt. The high nickel content of the alloy is believed to provide needle 10 with its MR compatibility. Needle 10 formed of alloy C-276 provides sufficient artifact to allow localization of a needle in the MR image.

In many instances, for example, when head or neck lesions are to be biopsied, thin section imaging is needed. When sections are thin and lesions are small, artifacts from standard biopsy needles distort the image so severely that accurate localization and needle placement cannot be made. Further, new field echo imaging techniques with narrow flip angles tend to accentuate magnetic susceptibility artifacts of all needle types due to increased $T2^*$ sensitivity of gradient refocusing techniques.

The nickel content of needle 10 provides less artifact on both spin echo and gradient echo sequences which is ideal for MR-guided aspiration cytology of head and neck lesions using high resolution, thin section images. Alloy C-276 provides sufficient artifact for localization without obscuring underlying anatomy.

Experiments performed with needles such as needle 10, which contains 49% nickel, have resulted in clear images with good needle visualization. In one experiment the needle was suspended in a phantom made of 2.5 mM nickel chloride solution for imaging. The MR scans were performed on the 0.3 Tesla MR Scanner, Fonar Beta 3000 whole body imager available from Fonar Corporation of Melville, New York, as well as on 0.5 Tesla and 1.5 Tesla instruments available from the Picker Corporation of Highland Heights, Ohio. With one signal excitation on a 256 x 256 matrix, two dimensional Fourier transformation (2D-FT) images were obtained. Field-echo sequences using a reduced flip angle of 30° and a standard spin-echo sequences were employed with an echo time (TE) of 14 milli-seconds and a repetition time (TR) of 400 milli-seconds.

Similar experiments were done with needles having 12% nickel and 9% nickel. The needle having 9% nickel content produced artifacts which tended to obscure accurate needle tip localization. The needle having 12% nickel content, although producing less artifact than the 9% nickel needle, still produced artifacts that tended to obscure the needle tip. With both of these needles, the artifacts were increased on the field-echo sequences relative to spin-echo sequences. The needle with 49% nickel, on both spin-echo and field echo sequences produced fewer artifacts. With that needle, the needle tip was not obscured, surprisingly enough artifact was present to allow tip visualization.

In a second study, five different alloys with concentrations of nickel ranging from 9-73% were studied. The chromium concentration in all the alloys was approximately 20%. The alloys were in tube form. All scans were done using the aforementioned Fonar or Picker scanners. Each sample was imaged both with a spin echo (SE/300/30) sequence and a field echo (FE/300/201 90°) sequence. Slices were 5 mm thick with a field view of 25.6cm. It was found that in all cases the distortions produced in field echo image were greater than in the corresponding spin echo image. A dramatic reduction in artifact occurred when the nickel concentration was increased from 9% to 35%. The magnitude of the artifact progressively decreased as the nickel concentration increased, however, it was difficult to discern any artifact difference when the higher nickel concentrations (60% - 73%) were compared.

The increased nickel content of needle 10 results in reduced artifact in part because the nickel reduces the ferromagnetic properties of the iron in the alloy. The reduced ferromagnetic properties of the iron in the alloy reduce its magnetic susceptibility so that it is closer to the magnetic susceptibility of the surrounding tissue.

Abrupt local changes in induced magnetic field susceptibility result in magnetic field inhomogeneities which interfere with gradient fields used in imaging. The result is local geometric image distortions oriented along the read gradient. Alterations in image intensity also occur with a similar orientation. A combination of these geometric brightness distortions can completely obscure underlying anatomy and pathology. The increased nickel content reduces these effects.

The lack of the 180° refocusing pulse allowing an extremely short TE and TR with gradient refocusing techniques also means that the imaging sequences are generally much more sensitive to magnetic inhomogeneities and susceptibility differences. Therefore, the magnetic susceptibility artifacts produced by all the needles are accentuated by using gradient refocusing techniques. Because of the high speed of field-echo imaging, this technique is useful for MR-guided biopsies. Thus, MR needles and other devices with minimal differences in susceptibility are especially valuable. The overall trend toward higher resolution, thinner section imaging makes needles and other devices with smaller artifacts increasingly important for MR imaging and biopsies throughout the body.

Although the particular dimensions and tip of the biopsy needle used will be a function of where the lesion to be biopsied is, one embodiment of the needle of the present invention uses a Chiba tip. The needle is a 22 gauge needle, 5 -20 centimeters long, having an outer diameter of about .071 centimeters, a wall thickness of about 0.010 centimeters, and an inner diameter of about 0.0508 centimeters. The degree of needle tip bevel level, annealing, or length has no significant effect on image artifacts. A minimal increase in artifacts occur when needle diameter is increased.

A needle such as needle 10 may be used with winged infusion sets. Winged infusion sets can be used during MRI and increasingly more patients are being sent for MR examinations with winged infusion sets in place.

Studies have been made in the presence of MR comparing a winged infusion set employing a standard stainless steel needle to a winged infusing set employing a needle formed of alloy C-276. The contrasted needles differed in gauge but were of similar length and configuration.

Imaging studies were performed on a 0.3 Tesla hybrid MR unit (Fonar Beta 3000M, Melville, New York) and superconducting MR units at 0.5 and 1.5 Tesla (Vista, Picker Corporation, Highland Heights, Ohio). Standard spin-echo as well as gradient echo images were used on all instruments.

In one experiment, the infusion set needle was suspended by a thread using a known technique. The amount of deflection was recorded as the needle was moved to various distances from the magnet bore of the MR instruments. The deflection force was calculated. In another experiment, the infusion sets were attached to a volunteer and imaged using the 0.3 Tesla MR instrument. Spin-echo (SE/500/30) and field echo (FE/100/10/20) pulse sequences were obtained through the needle and image quality was compared.

With the standard stainless steel needle infusion set, the force of deflection increased with proximity to the magnet for a given field strength. This phenomenon also increased with increasing field strength. However, with the infusion set employing a needle formed of alloy C-276, no measurable deflection from any distance or any field strength was measured. The deflection that occurs in MRI instruments with conventional stainless steel winged infusion needles could present a problem for the patient in the MR environment. Situations where winged infusion set are used often involve precarious placement of the needle in a vessel where magnetic field induced motion could dislodge the needle tip or result in infiltration.

The studies done on the infusion set needle also involved comparisons of the artifact produced by the standard stainless steel needle and the needle of alloy C-276. The results of these studies were similar to those done with the guided aspiration biopsy needle. While significant artifacts were present in the spin echo images for the stainless steel infusion set at 0.3 Tesla and while said artifact was even more pronounced on the field echo images, images obtained with infusion set 100 showed no significant artifact on the spin echo or field sequences.

The use of the inventive MR compatible infusion set greatly reduces or eliminates deflection effects in the magnetic field and is thus valuable in such situations requiring winged infusion devices in the MR scanning environment.

Other implements formed of alloy C-276 may be useful when MR imaging is performed. As the use of MR imaging expands, use of MRI compatable devices will also become more important.

**Claims**

1. An implement to be used with high resolution magnetic resonance imaging which is formed of an alloy containing nickel, characterised in that the alloy comprises at least 35% nickel by weight.

2. An implement as claimed in claim 1 characterised in that it is an aspiration biopsy needle.

3. An implement as claimed in claim 1 or claim 2 characterised in that the alloy comprises at least 49% nickel by weight.

4. An implement as claimed in any one of the preceding claims characterised in that the alloy consists essential of : about 49% by weight nickel; about 15.00 - 17.00% by weight molybdenum; about 14.50 - 16.50% by weight chromium; about 4.00 - 7.00% by weight iron; about 3.00 - 4.50% by weight tungsten; up to about 0.08% by weight carbon; up to about 1.00% by weight magnesium; up to about 1.00% by weight silicon; up to about 2.50% by weight cobalt.

FIG.1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | WO-A-8 704 080 (LONGMORE) <br> * Page 1, lines 3-13 * | 1 | A 61 B 17/34 <br> G 01 N 24/02 <br> A 61 B 10/00 |
| A | US-A-3 342 175 (BULLOCH) <br> * Column 1, line 67 - column 2, line 8; figure 2 * | 2 | |
| A | US-A-4 088 478 (CULLING) <br> * Abstract * | 4 | |

TECHNICAL FIELDS SEARCHED (Int. Cl.4)

A 61 F
A 61 B
A 61 M
G 01 R
C 22 C

The present search report has been drawn up for all claims

| Place of search | Date of completion of the search | Examiner |
|---|---|---|
| THE HAGUE | 26-04-1989 | MOERS R.J. |